# EUROPÄISCHE PATENTSCHRIFT

(11) **EP 1 916 978 B1**
(45) Veröffentlichungstag und Bekanntmachung des Hinweises auf die Patenterteilung: **17.02.2016**
(21) Anmeldenummer: 06776968.7
(22) Anmeldetag: 18.08.2006
(51) Int. Cl.: A61F 9/01

(54) **VERFAHREN ZUR ERMITTLUNG VON STEUERINFORMATIONEN FÜR DIE PHOTOREFRAKTIVE HORNHAUTCHIRURGIE SOWIE VERFAHREN ZUR BEREITSTELLUNG HIERBEI BENÖTIGTER KORREKTURINFORMATIONEN**
METHOD FOR DETERMINING CONTROL INFORMATION FOR PHOTOREFRACTIVE CORNEA SURGERY AND METHOD FOR PREPARING NECESSARY CORRECTION INFORMATION
PROCEDE POUR DETERMINER DES INFORMATIONS DE COMMANDE POUR LA CHIRURGIE PHOTOREFRACTIVE CORNEENNE ET PROCEDE POUR PREPARER DES INFORMATIONS CORRECTIVES NECESSAIRES

(30) Priorität: 19.08.2005 DE 102005039367
(43) Veröffentlichungstag der Anmeldung: 07.05.2008
(73) Patentinhaber: WaveLight GmbH, 91058 Erlangen (DE)
(72) Erfinder: MROCHEN, Michael, CH-8193 Eglisau (CH); BÜELER, Michael, CH-8048 Zürich (CH)
(74) Vertreter: Katérle, Axel
(86) Internationale Anmeldenummer: PCT/EP2006/008176
(87) Internationale Veröffentlichungsnummer: WO 2007/020105

(56) Entgegenhaltungen:
- US-A1- 2005 107 775

## Beschreibung

Die Erfindung betrifft das technische Gebiet der photorefraktiven Hornhautchirurgie, bei der Hornhautgewebe mittels Laserstrahlung mit dem Ziel der Behebung oder zumindest weitgehenden Minderung von Sehfehlern bearbeitet wird.

Die laserbasierte photorefraktive Hornhautchirurgie ist ein etabliertes Verfahren zur Korrektur von optischen Fehlern des Auges und damit zur Verbesserung der Sehkraft des Patienten. Insbesondere können mit diesem Verfahren Fehlsichtigkeiten niederer Ordnung behandelt werden, also zum Beispiel Myopie (Kurzsichtigkeit), Hyperopie (Weitsichtigkeit), Astigmatismus (zylindrische Fehlsichtigkeit), myoper Astigmatismus und hyperoper Astigmatismus. Ein bekanntes Verfahren der kornealen Laserchirurgie (Kornea = Hornhaut) ist die sogenannte LASIK (Laser In Situ Keratomileusis). Hierbei wird von der Hornhaut des zu behandelnden Auges zunächst ein Scheibchen bis auf einen geringen, als "Scharnier" dienenden Rest abgeschnitten. Dieses Scheibchen, das in der Fachwelt allgemein mit dem englischsprachigen Begriff Flap bezeichnet wird, wird sodann zur Seite geklappt, woraufhin mittels eingestrahlter Laserstrahlung Material inmitten der Hornhaut abgetragen wird. Der Materialabtrag erfolgt im wesentlichen im Stroma der Hornhaut, das ist der unter dem Hornhautepithel und der Bowman-Membran liegende Teil des Hornhautgewebes. Die Gewebeentfernung mittels Laserstrahlung wird auch als Ablation bezeichnet. Nach der Laserbehandlung wird das Scheibchen wieder auf seinen ursprünglichen Platz zurückgeklappt. Weil keine offene Wunde am Auge verbleibt, heilt der Flap in der Regel recht schnell wieder am darunterliegenden Hornhautgewebe an.

Die LASIK ist nicht die einzige bekannte Methode, bei der zur Behebung von Fehlsichtigkeiten Hornhautgewebe mittels Laserstrahlung abgetragen wird. Nur beispielhaft sei auf die sogenannte photorefraktive Keratektomie (PRK) als weiteren Vertreter einer derartigen Behandlungsmethode verwiesen. Die Erfindung ist generell bei beliebigen Methoden der kornealen Laserchirurgie anwendbar, bei denen Hornhautgewebe mittels Laserstrahlung ablatiert wird; eine Beschränkung auf bestimmte Techniken ist keinesfalls beabsichtigt.

Vor dem Gewebeabtrag muss ein sogenanntes Ablationsprofil erstellt werden. Dieses gibt an, wie viel Gewebe an welcher Stelle des kornealen Behandlungsgebiets abgetragen werden soll. Das Ablationsprofil kann somit als eine Höhenkarte verstanden werden, die die Höhe des erforderlichen Gewebeabtrags an den verschiedenen Stellen des Behandlungsgebiets festlegt. Beispielsweise kann das Ablationsprofil in Form einer zweidimensionalen Matrix erstellt werden, deren Matrixelemente die einzelnen Höhenwerte angeben.

Eine bekannte Technik zur Ermittlung des Ablationsprofils beruht auf der Ermittlung sogenannter Wellenfrontaberrationen des Auges. Die optische Abbildung im Auge wird nicht nur durch sphärische und zylindrische Fehler beeinträchtigt, sondern auch durch Abbildungsfehler höherer Ordnung. Wellenfrontmessungen, wie sie beispielsweise mit einem Hartmann-Shack-Sensor oder einem Tscherning-Sensor durchgeführt werden können, gestatten die Messung auch höherer Aberrationen des Auges. Für weitere Informationen hinsichtlich der verschiedenen durch die Hornhaut und das intraokulare Abbildungssystem bedingten Aberrationen des Auges und deren Messung mittels Wellenfrontsensoren kann beispielsweise auf den Aufsatz von W. Wesemann: "Optische und physiologische Grenzen der wellenfrontgesteuerten Hornhautchirurgie" in Der Ophthalmologe, 5, 2004, Seiten 521-537, verwiesen werden. Ergebnis der Aberrationsmessung ist ein sogenanntes Wellenfrontaberrationsgebirge, das für verschiedene Pupillenorte jeweils einen Wert für die Wellenfrontaberration angibt. Je höher das "Gebirge" an einer Stelle ist, um so größer sind die Abbildungsverzerrungen an dem betreffenden Pupillenort. Das Wellenfrontaberrationsgebirge kann mathematisch beispielsweise mit Hilfe von Zernike-Polynomen oder Taylor-Polynomen approximiert werden. Aus dem Wellenfrontaberrationsgebirge lässt sich ein zugehöriges Ablationsprofil beispielsweise dadurch ableiten, dass iterativ eine angenommene Ablationstiefe so verändert wird, bis eine möglichst gute Angleichung der Laufzeiten von Lichtstrahlen an allen Orten der Hornhaut und damit eine möglichst gute Einebnung des Wellenfrontaberrationsgebirges erreicht ist.

Die auf Grundlage einer Vermessung der optischen Eigenschaften des Auges gewonnenen Ablationsprofile sind freilich mit dem Nachteil behaftet, dass sie oftmals nur theoretische Gültigkeit haben. In der Praxis beeinträchtigen verschiedenartige Störfaktoren während und nach der Behandlung die Genauigkeit der Fehlsichtigkeitskorrektur. So können beispielsweise periphere Reflektionsverluste beim Laserabtrag, post-operative Wundheilungsprozesse oder biomechanische Veränderungen der Hornhaut zu Abweichungen zwischen der angestrebten (theoretisch idealen) und der tatsächlich erreichten Formänderung der Hornhaut führen.

Es wurde deshalb vorgeschlagen, das Ablationsprofil mittels empirisch ermittelter Korrekturfaktoren zu modifizieren und die Laserbehandlung nach Maßgabe des so korrigierten Ablationsprofils durchzuführen. Eine mögliche Vorgehensweise dabei ist, die Korrekturfaktoren durch Mittelung der post-operativen Ergebnisse einer großen Anzahl von Patienten zu ermitteln. Die durch Vermessung des Auges gewonnenen, unkorrigierten Soll-Abtragsprofile einer Vielzahl von Patienten werden hierfür mit den sich nach Behandlung und abgeschlossener Heilung tatsächlich ergebenden Ist-Abtragsprofilen verglichen. Auf Basis dieser empirischen Daten wird sodann eine einheitliche Korrekturmatrix ermittelt, die gleiche Größe wie jene des Ablationsprofils besitzt. Jedes Matrixelement der Korrekturmatrix bezeichnet einen ortsabhängigen Korrekturfaktor für den entsprechenden Höhenwert der Ablationsmatrix. Die Korrekturfaktoren der Korrekturmatrix können beispielsweise als Multiplikatoren angegeben werden, mit denen die Höhenwerte der Abtragsmatrix elementweise zu multiplizieren sind, um zu einer korrigierten Abtrags- bzw. Ablationsmatrix zu gelangen.

An jenen Stellen der Hornhaut, wo der reale Abtrag (Ist-Wert) nach den empirischen Daten gut mit dem geplanten Abtrag (Soll-Wert) übereinstimmt, hat der Korrekturfaktor den Wert Eins, dort hingegen, wo in der Regel zu wenig abgetragen wird, steht ein Korrekturfaktor mit einem größeren Wert als Eins. Gegenwärtig in der Praxis eingesetzte Korrekturmatrizen können mit einer parabolischen Form verglichen werden. Vom Zentrum (das der Mitte der Hornhautebene entspricht) nehmen die Korrekturfaktoren zu den Matrixrändern hin entsprechend der Parabelform zu. Die Korrektur des Ablationsprofils erfolgt bei Verwendung solcher Korrekturmatrizen somit im geometrischen Raumbereich durch einfache Multiplikation der Matrixelemente der Korrekturmatrix mit denen der Ablationsmatrix.

Es hat sich gezeigt, dass insbesondere bei Augen mit stark irregulären Strukturen die so zu erreichende Kompensation der Störfaktoren oftmals ungenügend ist.

US 2005/107775 A1 lehrt die Verwendung einer Korrekturfunktion mit Tiefpasscharakteristik. Die Tiefpasscharakteristik soll post-operative Glättungsprozesse des Hornhautepithels kompensieren. Die Korrektur eines für ein zu behandelndes Auge ermittelten Ablationsprofils erfolgt im geometrischen Raumbereich durch iterative Faltung, bis ein korrigiertes Profil erhalten wird, das dem Faltungsprodukt des ursprünglichen Ablationsprofils mit der geometrischen Impulsantwortfunktion des Tiefpassfilters entspricht. Diese Methodik ist rechentechnisch äußerst aufwendig. Aufgabe der Erfindung ist es, einen Weg zur patientenspezifischen Korrektur eines Hornhautablationsprofils aufzuzeigen, der akzeptablen Rechenaufwand mit sich bringt und keine grundlegende Beschränkung auf die Kompensation bestimmter vorab spezifizierter Störeffekte beinhaltet.

Bei der Lösung dieser Aufgabe geht die Erfindung aus von einem Verfahren zur Ermittlung von Steuerinformationen für die Steuerung von auf die Hornhaut eines photorefraktiv zu behandelnden Auges eingestrahlter Laserstrahlung, wobei bei diesem Verfahren ein durch Vermessung der optischen Eigenschaften des zu behandelnden Auges gewonnenes Hornhautablationsprofil anhand von Korrekturinformationen korrigiert wird und die Steuerinformationen auf Grundlage des so erzeugten korrigierten Ablationsprofils gebildet werden.

Erfindungsgemäß ist dabei gemäß Anspruch 1 vorgesehen, dass das Ablationsprofil in ein Ortsfrequenzspektrum transformiert wird, dass sodann für verschiedene diskrete Spektralkomponenten des Ablationsprofils auf Grundlage gespeicherter Amplitudenkorrekturinformationen jeweils ein korrigierter Amplitudenwert ermittelt wird und dass zur Ermittlung des korrigierten Ablationsprofils anschließend das Ortsfrequenzspektrum mit den amplitudenkorrigierten Spektralkomponenten in den geometrischen Raumbereich zurücktransformiert wird, wobei die Amplitudenkorrekturinformationen für mehrere sich zumindest durch den Frequenzwert unterscheidende Ortsfrequenzen jeweils einen vorab ermittelten Zusammenhang zwischen Werten der Amplitude bei der betreffenden Ortsfrequenz und korrigierten Amplitudenwerten bei dieser Ortsfrequenz repräsentieren.

Die Erfindung ist nicht allein auf die Amplitudenkorrektur der Spektralkomponenten des Ortsfrequenzspektrums beschränkt. Sie ist ebenso zur spektralen Phasenkorrektur anwendbar. Dementsprechend kann bei einem Verfahren der gattungsgemäßen Art gemäß Anspruch 2 vorgesehen sein, dass das Ablationsprofil in ein Ortsfrequenzspektrum transformiert wird, dass sodann für verschiedene diskrete Spektralkomponenten des Ablationsprofils auf Grundlage gespeicherter Phasenkorrekturinformationen jeweils ein korrigierter Phasenwert ermittelt wird und dass zur Ermittlung des korrigierten Ablationsprofils anschließend das Ortsfrequenzspektrum mit den phasenkorrigierten Spektralkomponenten in den geometrischen Raumbereich zurücktransformiert wird, wobei die Phasenkorrekturinformationen für mehrere sich zumindest durch den Frequenzwert unterscheidende Ortsfrequenzen jeweils einen vorab ermittelten Zusammenhang zwischen Werten der Phase bei der betreffenden Ortsfrequenz und korrigierten Phasenwerten bei dieser Ortsfrequenz repräsentieren.

Alternativ oder zusätzlich zur Amplituden- und Phasenkorrektur kann die Erfindung gemäß Anspruch 3 den Realteil oder/und gemäß Anspruch 4 den Imaginärteil eines oder mehrerer Spektralkomponenten des Ortsfrequenzspektrums (bei komplexer Darstellung) korrigieren. Dementsprechend kann erfindungsgemäß für verschiedene diskrete Spektralkomponenten des Ablationsprofils auf Grundlage gespeicherter Realteil- oder/und Imaginärteilkorrekturinformationen jeweils ein korrigierter Realteilwert bzw. ein korrigierter Imaginärteil ermittelt werden. Zur Ermittlung des korrigierten Ablationsprofils wird anschließend das Ortsfrequenzspektrum mit den realteil- oder/und den imaginärteilkorrigierten Spektralkomponenten in den geometrischen Raumbereich zurücktransformiert. Die Realteilkorrekturinformationen repräsentieren dabei für mehrere sich zumindest durch den Frequenzwert unterscheidende Ortsfrequenzen jeweils einen vorab ermittelten Zusammenhang zwischen Werten des Realteils bei der betreffenden Ortsfrequenz und korrigierten Realteilwerten bei dieser Ortsfrequenz. Die Imaginärteilkorrekturinformationen repräsentieren entsprechende Zusammenhänge für den spektralen Imaginärteil.

Gemäß der Erfindung werden somit ein oder mehrere Parameter (Amplitude, Phase, Realteil, Imaginärteil) verschiedener diskreter Spektralkomponenten des Ortsfrequenzspektrums anhand vorab empirisch ermittelter Zusammenhänge korrigiert. Nachfolgend werden nur noch die Parameter Amplitude und Phase ausdrücklich erwähnt; es versteht sich jedoch, dass die diesbezüglichen Aussagen gleichermaßen auf Realteil und Imaginärteil der Spektralkomponenten übertragbar sind.

Die Erfindung beruht auf der Beobachtung, dass verschiedene räumliche Frequenzanteile innerhalb eines Ablationsprofils in unterschiedlicher Form auf die Hornhaut übertragen werden. Einige Spektralkomponenten des Ablationsprofils werden mehr oder weniger stark gedämpft, während sich andere im wesentlichen ungedämpft oder unter Umständen sogar verstärkt im Ist-Abtragsprofil wiederfinden. Nicht nur die Amplitude der Spektralanteile kann sich jedoch bei der Übertragung des Abtragsprofils in die Hornhaut ändern, sondern auch die Phase. Mit den durch die Korrekturinformationen repräsentierten amplituden- und phasenbezogenen Funktionszusammenhängen kann diesen Amplituden- und Phasenänderungen Rechnung getragen werden. Für eine Vielzahl diskreter Spektralkomponenten des Ablationsprofils kann dabei mit Hilfe dieser Zusammenhänge jeweils eine amplituden- oder/und phasenkorrigierte Spektralkomponente ermittelt werden. Durch Rücktransformation des Spektrums mit den amplituden- oder/und phasenkorrigierten Spektralkomponenten in den Raumbereich wird ein korrigiertes Ablationsprofil erhalten, das nach Operation und nach abgeschlossener Wundheilung und sonstigen post-operativen Übergangsvorgängen mit guter Genauigkeit zu dem gewünschten Soll-Ablationsprofil führt.

Die Erzeugung der Amplituden- oder/und Phasenkorrekturinformationen erfolgt vorzugsweise in der Weise, dass eine Vielzahl von Soll-Ablationsprofilen und eine Vielzahl zugehöriger Ist-Ablationsprofile jeweils in ein Ortsfrequenzspektrum transformiert werden, dass anhand der Soll- und der Ist-Ortsfrequenzspektren für mehrere sich hinsichtlich Frequenzwert oder/und Raumrichtung unterscheidende diskrete Ortsfrequenzen jeweils ein funktionaler Zusammenhang für die Abhängigkeit der Ist-Amplitude oder/und der Ist-Phase bei der betreffenden Ortsfrequenz von der Soll-Amplitude bzw. der Soll-Phase bei dieser Ortsfrequenz ermittelt wird und dass die Amplituden- oder/und Phasenkorrekturinformationen von Daten gebildet werden, welche die ermittelten funktionalen Zusammenhänge beschreiben.

Diese Methode greift im Unterschied zu der Vorgehensweise gemäß US 2005/107775 A1 nicht auf ein vorab festgelegtes Übertragungsmodell zurück, sondern ermittelt anhand einer vorhandenen Datenbasis von Soll-Ablationsprofilen und zugehörigen Ist-Ablationsprofilen für verschiedene diskrete Ortsfrequenzen jeweils individuell eine amplitudenbezogene oder/und eine phasenbezogene Übertragungsfunktion. In US 2005/107775 A1 wird ein Butterworth-Tiefpass als Übertragungsmodell verwendet. Dieses Model ist amplituden- und phasenunabhängig; bei jeder Ortsfrequenz wird das durch Vermessung gewonnene Ablationsprofil eines Patienten mit einem amplituden- und phasenunabhängigen, nur vom betreffenden Frequenzwert abhängigen Faktor gewichtet. Die erfindungsgemäße Herangehensweise dagegen hebt die Beschränkung auf amplituden- und phasenunabhängige Wichtungsfaktoren auf. Drückt die vorhandene Datenbasis eine nichtlineare Abhängigkeit der Ist- von der Soll-Amplitude oder der Ist- von der Soll-Phase bei einer oder mehreren Ortsfrequenzen aus, so kann dies durch die Funktionszusammenhänge widergespiegelt werden.

Darüber hinaus gestattet die erfindungsgemäße Vorgehensweise die Berücksichtigung von Frequenzkopplungen, ein in US 2005/107775 A1 nicht berücksichtigter Gesichtspunkt. Es hat sich anhand von Untersuchungen gezeigt, dass die Amplituden- oder/und Phasenübertragungsfunktion bei einer Ortsfrequenz auch abhängen kann von der Amplitude bzw. Phase bei einer oder mehreren anderen Ortsfrequenzen. Anders ausgedrückt können die verschiedenen Spektralkomponenten eines Soll-Ablationsprofils wechselseitig Einfluss auf das sich post-operativ einstellende Ist-Ablationsprofil nehmen. Bei der Ermittlung der funktionalen Zusammenhänge empfiehlt es sich daher, die Abhängigkeit der Ist-Amplitude oder/und der Ist-Phase mindestens einer Ortsfrequenz von der Soll-Amplitude bzw. der Soll-Phase mindestens einer anderen Ortsfrequenz zu untersuchen, derart, dass mindestens ein Teil der funktionalen Zusammenhänge die Ist-Amplitude oder/und die Ist-Phase bei der jeweils betreffenden Ortsfrequenz auch in Abhängigkeit von der Soll-Amplitude bzw. der Soll-Phase bei mindestens einer anderen Ortsfrequenz angibt.

Die Amplituden- oder/und Phasenkorrekturinformationen können in verschiedener Weise implementiert werden. Eine Möglichkeit besteht in einer tabellarischen Darstellung, eine andere in einer Darstellung in Form einer oder mehrerer mathematischer Formeln. Als Ausgabe können die Amplituden- oder/und Phasenkorrekturinformationen unmittelbar die korrigierten Amplituden- und Phasenwerte liefern. Alternativ können die Amplituden- oder/und Phasenkorrekturinformationen als Ausgabe lediglich Korrekturwerte liefern, mit denen die Spektralkomponenten des Ablationsprofils noch multiplikativ oder additiv zu verknüpfen sind.

Verschiedene Spektralkomponenten des Ablationsprofils können gemäß einer Ausführungsform individuell auf Grundlage eines jeweils zugeordneten Funktionszusammenhangs korrigiert werden. Im Fall eines raumrichtungsabhängigen Ortsfrequenzspektrums kann es dann insbesondere dazu kommen, dass Spektralanteile gleichen Frequenzwerts, jedoch unterschiedlicher Raumrichtung unterschiedlich korrigiert werden. Auf diese Weise ist eine sehr feine, frequenz- und raumrichtungsaufgelöste Korrektur des Ablationsprofils möglich. Bei Bedarf können die Korrekturinformationen sogar für alle Spektralkomponenten des Ortsfrequenzspektrums des Ablationsprofils je einen amplitudenbezogenen oder/und je einen phasenbezogenen Funktionszusammenhang für die spektrale Amplitude bzw. Phase umfassen, um eine individuelle Korrektur aller Spektralkomponenten zu gestatten.

Es ist ebenso vorstellbar, für eine Gruppe von Spektralkomponenten gleichen Frequenzwerts, jedoch unterschiedlicher Raumrichtung des Ablationsprofils eine gemeinsame amplituden- oder/und phasenkorrigierte Spektralkomponente auf Grundlage der Amplituden- oder/und Phasenkorrekturinformationen zu ermitteln. Bei dieser Variante werden einzelne Spektralkomponenten gleichen Frequenzwerts nicht unterschiedlich korrigiert. Stattdessen wird eine gemeinsame amplituden- oder/und phasenkorrigierte Spektralkomponente ermittelt, die für alle Raumrichtungen der Gruppe gleich ist. Beispielsweise können hierzu die Amplituden oder/und Phasen der Spektralkomponenten der Gruppe gemittelt werden und die gemeinsame amplituden- oder/und phasenkorrigierte Spektralkomponente abhängig von dem Amplituden- bzw. Phasenmittelwert ermittelt werden.

Wurden bei der Ermittlung der Korrekturinformationen mögliche Frequenzkopplungen berücksichtigt, so kann mindestens einer der durch die Amplitudenkorrekturinformationen repräsentierten Zusammenhänge die korrigierten Amplitudenwerte bei der betreffenden Ortsfrequenz auch abhängig vom Wert der Amplitude bei mindestens einer anderen Ortsfrequenz angeben. Gleichermaßen kann mindestens einer der durch die Phasenkorrekturinformationen repräsentierten Zusammenhänge die korrigierten Phasenwerte bei der betreffenden Ortsfrequenz auch abhängig vom Wert der Phase bei mindestens einer anderen Ortsfrequenz angeben.

Zur Spektraltransformation des Ablationsprofils eines zu behandelnden Auges empfiehlt sich die Fourier-Transformation. Für eine frequenzdiskrete Darstellung des Ortsfrequenzspektrums des Ablationsprofils genügt die diskrete Fourier-Transformation, wobei für eine hohe rechentechnische Effizienz auf Methoden der schnellen diskreten Fourier-Transformation zurückgegriffen werden kann. Für die Rücktransformation des korrigierten Ortsfrequenzspektrums kann dementsprechend die inverse diskrete Fourier-Transformation, insbesondere die schnelle inverse diskrete Fourier-Transformation, eingesetzt werden. Die Fourier-Transformation und ihr inverser Prozess sind in der Fachwelt gängige Methoden zur Ermittlung eines Frequenzspektrums (insbesondere eines diskreten Frequenzspektrums) aus einem Zeit- oder Raumsignal (das Ablationsprofil des zu behandelnden Auges stellt tatsächlich ein zweidimensionales räumliches Signal dar). Auf die Erläuterung der zugrundeliegenden Mathematik der Fourier-Transformation und ihrer Inversion kann deshalb hier verzichtet werden.

Für die gesonderte Ermittlung der Amplitude und der Phase der verschiedenen Spektralkomponenten des Ortsfrequenzspektrums des Ablationsprofils empfiehlt sich insbesondere die komplexe Fourier-Analyse im komplexen Zahlenraum. Auch diesbezüglich sind die mathematischen Grundlagen dem einschlägigen Fachmann hinlänglich vertraut.

Es versteht sich, dass die Fourier-Analyse nicht die einzige Methode zur spektralen Untersuchung des Ablationsprofils ist. Denkbar sind selbstverständlich auch andere Ortsfrequenztransformationen. Nur als Beispiel sei auf die diskrete Kosinustransformation verwiesen, die ein räumliches Signal durch Kosinusreihen approximiert.

Die Erfindung wird nachfolgend anhand der beigefügten Zeichnungen weiter erläutert. Es stellen dar:
Figur 1 schematisch ein Ausführungsbeispiel einer Einrichtung zur Durchführung einer photorefraktiven Hornhautchirurgie des Auges,
Figur 2 Beispiele für Fittingfunktionen zur funktionalen Darstellung des spektralen Amplitudenübertragungsverhaltens bei der Hornhautbehandlung für verschiedene Ortsfrequenzen,
Figur 3 ein schematisches Prinzipbild zur Erläuterung einer gruppenweisen Amplitudenkorrektur der Spektralkomponenten eines Hornhautablationsprofils und
Figur 4 ein schematisches Prinzipbild zur Erläuterung einer individuellen Korrektur der Spektralkomponenten eines Hornhautablationsprofils.

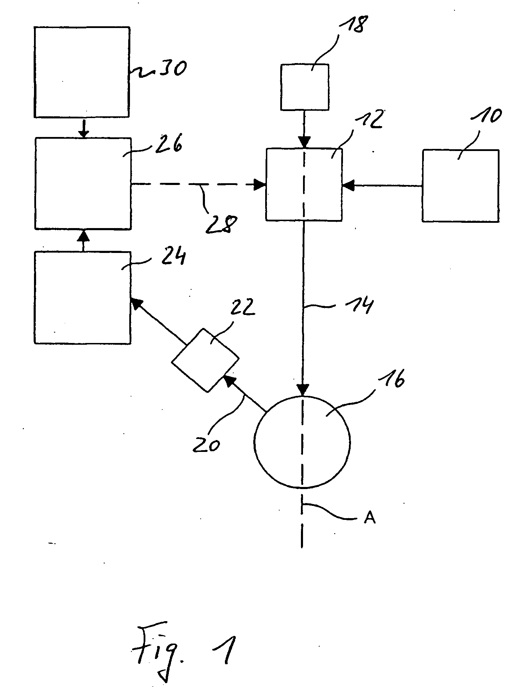

Die in Figur 1 gezeigte Einrichtung zur Durchführung einer photorefraktiven Hornhautbehandlung umfasst einen Laser 10, welcher die für die Photoablation benötigte Laserstrahlung bereitstellt. Die Photoablation kann auf der Hornhautoberfläche durchgeführt werden oder/und intra-stromal, also im Hornhautstroma. Als Laser 10 kommt für die Photoablation insbesondere ein Excimerlaser mit einer Emissionswellenlänge von beispielsweise 193nm in Betracht. Alternativ kann beispielsweise ein Er:YAG-Festkörperlaser mit einer Emissionswellenlänge von 2,94µm oder ein UV-Festkörperlaser (z.B. Nd:YAG mit 213nm) verwendet werden.

Die Laserstrahlung wird mittels eines galvanometrischen Abtasters (Scanner) 12 umgelenkt; der umgelenkte Laserstrahl - mit 14 bezeichnet - wird anschließend auf die Hornhaut eines zu behandelnden Auges 16 gerichtet. Koaxial zu dem Laserstrahl 14 wird ein weiterer Strahl auf das Auge 16 gerichtet. Dieser weitere Strahl stammt von einer sogenannten Positionierlichtquelle 18 und definiert eine im Raum ortsfeste Bezugsachse A.

Während der Operation bewegt sich das Auge 16 im Regelfall relativ zur Achse A. Zur Nachführung des Bearbeitungsstrahls 14 bei derartigen Augenbewegungen wird das Auge in nicht näher dargestellter Weise mit Infrarotstrahlung beleuchtet. Die mit 20 bezeichnete Bildstrahlung erzeugt in einer CCD- oder CMOS-Kamera 22 Bilder, die in einer nachgeschalteten Bildverarbeitungseinrichtung 24 elektronisch verarbeitet werden. Das Ergebnis der Bildverarbeitung wird in eine Rechen- und Steuereinheit 26 eingegeben, welche die Bildauswertung sowie die Steuerung des Abtasters 12 übernimmt. Die Rechen- und Steuereinheit 26 gibt hierzu ein geeignetes Stellsignal 28 an den Abtaster 12 aus. Dieses Stellsignal bewirkt eine derartige Lenkung des Bearbeitungsstrahls 14, dass ein zuvor für das zu behandelnde Auge 16 ermitteltes Ablationsprofil abgearbeitet wird. Die optischen Fehler des Auges 16 können so durch Photoablation von Hornhautgewebe korrigiert werden. Das in vorstehendem Sinne abgearbeitete Ablationsprofil ist ein korrigiertes Ablationsprofil, das aus einem durch Messung der optischen Eigenschaften des Auges 16 gewonnenen ursprünglichen Ablationsprofil und einer anschließenden Korrektur desselben hervorging.

Das ursprüngliche Ablationsprofil kann beispielsweise aus einer ortsaufgelösten Messung der Wellenfrontaberration des Auges 16 mittels eines Video-Aberroskops (nicht näher dargestellt) ermittelt werden. Für nähere Einzelheiten einer solchen Wellenfrontaberrationsmessung und der Ermittlung eines Ablationsprofils aus dem gemessenen Wellenfrontaberrationsgebirge wird auf DE 100 22 995 C2 verwiesen. Die Rechen- und Steuereinheit 26 kann dazu eingerichtet sein, die für die Berechnung des Wellenfrontaberrationsgebirges und des Ablationsprofils erforderlichen Rechenoperationen durchzuführen. Es ist ebenso möglich, dass das ursprüngliche Ablationsprofil mittels einer gesonderten Mess- und Rechenanordnung gewonnen wird und der Rechen- und Steuereinheit 26 lediglich das fertige Ablationsprofil zugeführt wird.

Ein elektronischer Speicher 30 enthält die benötigten Korrekturinformationen für die Korrektur des aus der Wellenfrontaberrationsmessung ermittelten ursprünglichen Ablationsprofils. Die Korrektur ist erforderlich, weil das sich post-operativ einstellende Ablationsprofil in der Regel von dem durch optische Vermessung des Auges 16 gewonnenen, theoretischen Soll-Ablationsprofil abweicht. Die Abweichungen können verschiedene Ursachen haben. Zum einen kann der post-operative Wundheilungsverlauf zu Veränderungen der Hornhaut führen. Beispielsweise kann durch Epithelglättung ein Tiefpassfiltereffekt hervorgerufen werden, wie in US 2005/107775 A1 erläutert. Der Wundheilungsverlauf kann nicht nur von den biologischen, insbesondere geweblichen Eigenschaften des jeweiligen Patienten abhängen, sondern auch vom verwendeten Lasersystem. Als weitere Einflussfaktoren, die zur Abweichung des sich letztlich ergebenden Ist-Ablationsprofils vom gewünschten Soll-Ablationsprofil führen können, sind optische Verluste während der Laserbehandlung zu nennen, insbesondere Reflexionsverluste.

Aufgrund der Vielzahl unterschiedlicher Störfaktoren, die zudem von Lasersystem zu Lasersystem und von Patient zu Patient unterschiedlich sein können, ist es in der Regel nicht möglich, allgemein gültige Daten oder Algorithmen für die Korrektur des Soll-Ablationsprofils anzugeben. Vielmehr müssen entsprechende Korrekturinformationen empirisch (experimentell) für das jeweils verwendete Lasersystem ermittelt werden, wobei die benötigte Datenbasis die Soll- und Ist-Ablationsprofile einer Vielzahl mit dem betreffenden Lasersystem behandelter Patienten umfasst. Es versteht sich, dass die Datenbasis mit den Daten neuerer Patienten fortwährend oder in regelmäßigen Abständen aktualisiert werden kann, um die Korrekturinformationen gegebenenfalls anzupassen.

Die im Speicher 30 abgelegten Korrekturinformationen repräsentieren Korrekturfunktionen für die spektrale Amplitude oder/und Phase für eine Mehrzahl verschiedener diskreter Ortsfrequenzen. Im folgenden wird der Übersichtlichkeit halber nur auf die spektrale Amplitudenkorrektur eingegangen, die Phasenkorrektur dagegen nicht weiter erläutert. Es ist jedoch ausdrücklich darauf hinzuweisen, dass die Anmerkungen zur Amplitudenkorrektur entsprechend für die spektrale Phasenkorrektur gelten.

Die durch die Korrekturinformationen repräsentierten Amplitudenkorrekturfunktionen stellen einen empirisch ermittelten Zusammenhang zwischen der Amplitude verschiedener Spektralanteile des sich post-operativ einstellenden Ablationsprofils (Ist-Amplitude) und der Amplitude der entsprechenden Spektralanteile des durch optische Vermessung des Auges 16 gewonnenen Soll-Ablationsprofils (Soll-Amplitude) dar. Jeder dieser Zusammenhänge beschreibt die Abhängigkeit der Ist-Amplitude bei einer bestimmten Ortsfrequenz abhängig zumindest von der Soll-Amplitude bei dieser Ortsfrequenz. In einer möglichen Weiterbildung kann zumindest ein Teil der Amplitudenkorrekturfunktionen die Ist-Amplitude der betreffenden Ortsfrequenz auch in Abhängigkeit der Soll-Amplitude bei einer oder mehreren anderen Ortsfrequenzen beschreiben. Auf diese Weise können Frequenzkopplungen bei der Übertragung des Soll-Ablationsprofils in das Ist-Profil berücksichtigt werden.

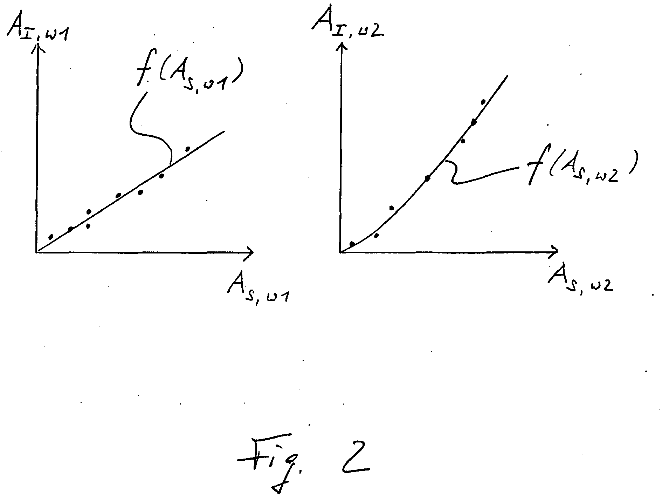

Für die Erläuterung der Vorgehensweise bei der Ermittlung der Amplitudenkorrekturfunktionen wird nun auf Figur 2 verwiesen. Dort sind zwei beispielhafte Diagramme gezeigt, die für zwei verschiedene Ortsfrequenzen ω₁, ω₂ die Wertepaare von Ist-Amplitude und Soll-Amplitude einer Vielzahl von Patienten bei der betreffenden Ortsfrequenz ω₁ bzw. ω₂ enthalten. Jedes Wertepaar ist in den Diagrammen durch einen Punkt angedeutet. A_{S}, ω₁ und A_{S}, ω₂ bezeichnen dabei die Soll-Amplitude bei der Frequenz ω₁ bzw. ω₂, wohingegen A_{I}, ω₁ und A_{I}, ω₂ die Ist-Amplitude bei der betreffenden Frequenz angeben. Die Soll- und Ist-Amplituden werden erhalten, indem das prä-operative Soll-Ablationsprofil und das post-operative Ist-Ablationsprofil jedes einzelnen Patienten der Datenbasis in den Ortsfrequenzbereich transformiert werden und aus beiden Spektren der Amplitudenwert bei der entsprechenden Spektrallinie gelesen wird. Die Spektraltransformation in den Ortsfrequenzbereich erfolgt vorzugsweise mittels diskreter Fourier-Transformation. Man erhält so für jedes Ablationsprofil ein matrixförmiges Ortsfrequenzspektrum, dessen Matrixelemente die Amplitude einer jeweiligen Spektrallinie des Spektrums angeben. (Bei Anwendung der komplexen Fourier-Transformation kann zusätzlich ein Phasenlinienspektrum ermittelt werden.) Die verschiedenen diskreten Spektrallinien des Ortsfrequenzspektrums sind durch Frequenzwert und Raumrichtung bestimmt. Nachdem das Ablationsprofil ein zweidimensionales Profil ist, das in einem X, Y-Koordinatensystem dargestellt werden kann, können die in dem Ablationsprofil enthaltenen Ortsfrequenzen raumrichtungsabhängig unterschiedliche Amplitude oder/und Phase haben.

Für jeden Patienten aus der Datenbasis werden somit zwei Amplitudenlinien-Spektren erhalten, nämlich das Ist-Spektrum und das Soll-Spektrum.

Mit den Soll- und Ist-Spektren der verschiedenen Patienten der Datenbasis steht folglich für jede von mehreren sich hinsichtlich Frequenzwert oder/und Raumrichtung unterscheidenden Ortsfrequenzen eine Sammlung von Wertepaaren von spektraler Soll-Amplitude und spektraler Ist-Amplitude bei der jeweils betreffenden Ortsfrequenz zur Verfügung. Jedes der Wertepaare stammt dabei von einem der Patienten. Die Punkte in den beiden Diagrammen der Figur 2 stellen jeweils eine solche Sammlung dar.

In einer anschließenden Phase wird nun versucht, die Abhängigkeit der Ist-Amplitude von der Soll-Amplitude bei der betreffenden Ortsfrequenz funktional zu beschreiben. Dies erfolgt in einem sogenannten Fitting-Verfahren, bei dem mit einer Fitting-Funktion die Verteilung der verschiedenen Wertepaare approximiert wird. In Figur 2 sind zwei verschiedene Beispiele einer solchen Fitting-Funktion gezeigt. Im Fall des linken Diagramms handelt es sich um eine lineare Fitting-Funktion (bezeichnet mit f(A_{S, ω1})), während im Fall des rechten Diagramms eine nichtlineare Fitting-Funktion, nämlich konkret eine quadratische Fitting-Funktion gezeigt ist (bezeichnet mit f(A_{S, ω2})). Es versteht sich, dass je nach Verteilung der Wertepaare von Ist-Amplitude und Soll-Amplitude völlig unterschiedliche Typen von Fitting-Funktionen verwendet werden können. Lineare und quadratische Fitting-Funktionen sind nur als bloße Beispiele zu betrachten; beliebige andere nichtlineare Fitting-Funktionen sind gleichermaßen verwendbar.

Solche Fitting-Funktionen werden für eine Vielzahl von Ortsfrequenzen ermittelt. Grundsätzlich kann für jede Spektrallinie eine gesonderte Fitting-Funktion ermittelt werden. Auch Spektrallinien gleichen Frequenzwerts, jedoch unterschiedlicher Raumrichtung kann so eine eigene Fitting-Funktion zugeordnet werden.

Gemäß einer abweichenden Ausführungsform wird zumindest ein Teil der Spektrallinien gruppenweise zusammengefasst, wobei jede Gruppe Spektrallinien gleichen (oder zumindest näherungsweise gleichen) Frequenzwerts, jedoch unterschiedlicher Raumrichtung enthält. Es wird hierbei eine Mittelung der Wertepaare von Soll-Amplitude und Ist-Amplitude jedes einzelnen Patienten über die verschiedenen Spektrallinien der Gruppe vorgenommen und für die gemittelten Wertepaare eine Fitting-Funktion gesucht. Sofern als Spektraltransformation die Fourier-Transformation verwendet wird und das Zentrum der sich ergebenden Spektralmatrizen der Ortsfrequenz Null zugeordnet ist, können Spektrallinien gleichen Frequenzwerts jedoch unterschiedlicher Raumrichtung in Ringen um das Matrixzentrum gefunden werden. Für jeden derartigen Frequenzring kann eine gemeinsame Fitting-Funktion ermittelt werden.

Die Rechen- und Steuereinheit 26 kann dazu eingerichtet sein, die Ermittlung der Fitting-Funktionen und gewünschtenfalls auch die Durchführung der Spektraltransformation der Ablationsprofile der Datenbasis vorzunehmen. Wenigstens ein Teil dieser Rechenoperationen kann jedoch auch von einer gesonderten Recheneinheit vorab durchgeführt werden. Jedenfalls sind im Speicher 30 geeignete Daten abgelegt, welche die Fitting-Funktionen beschreiben. Dies kann in Form eines Algorithmus oder in tabellarischer Form sein. Vorteilhaft an der Verwendung der Fitting-Funktionen ist, dass keine vorherige Festlegung auf ein bestimmtes Übertragungsmodell erforderlich ist, was eine Beschränkung auf bestimmte Störeffekte bedeuten würde. Gleichgültig, welcher Art die auftretenden Störeffekte sind, kann mit den Fitting-Funktionen eine gute Modellierung des tatsächlichen Übertragungsverhaltens von Soll-Ablationsprofil zu Ist-Ablationsprofil erreicht werden.

Es wird nun wieder auf Figur 1 verwiesen. Zur erfolgreichen Behandlung des Auges 16 ermittelt die Rechen- und Steuereinheit 26 aus dem durch optische Vermessung des Auges 16 gewonnen Soll-Ablationsprofil anhand der im Speicher 30 abgelegten Fitting-Funktionen ein korrigiertes Ablationsprofil und steuert den Abtaster 12 nach Maßgabe dieses korrigierten Profils. Für die Korrektur des Soll-Ablationsprofils führt die Rechen- und Steuereinheit 26 eine Spektraltransformation des Soll-Ablationsprofils in den Ortsfrequenzbereich durch und erhält so ein Soll-Ortsfrequenzspektrum für das Auge 16. Anschließend ermittelt die Rechen- und Steuereinheit 26 anhand der im Speicher 30 abgelegten Fitting-Funktionen korrigierte Amplitudenwerte für wenigstens einen Teil der Spektralkomponenten des Ortsfrequenzspektrums. (Alternativ oder zusätzlich kann die Rechen- und Steuereinheit 26 korrigierte Phasenwerte für wenigstens einen Teil der Spektralkomponenten ermitteln.) Mit den korrigierten Amplitudenwerten bildet die Rechen- und Steuereinheit 26 sodann ein korrigiertes Ortsfrequenzspektrum und transformiert dieses zurück in den geometrischen Raumbereich. Ergebnis ist das korrigierte Ablationsprofil, das zur Grundlage der Steuerung des Abtasters 12 gemacht wird.

Bei der spektralen Amplitudenkorrektur des Ablationsprofils kann jede Spektralkomponente des vorzugsweise in Matrixform vorliegenden Ortsfrequenzspektrums einzeln anhand einer jeweiligen Fitting-Funktion korrigiert werden. Wie zuvor erläutert, können einzelne Fitting-Funktionen jeweils einer Gruppe von Ortsfrequenzen gemeinsam zugeordnet sein. Dementsprechend kann die spektrale Korrektur des Ablationsprofils auch gruppenweise erfolgen, indem Gruppen von Spektralkomponenten gleichen Frequenzwerts, jedoch unterschiedlicher Raumrichtung des Ablationsprofils amplitudenmäßig gemittelt werden und der so ermittelte Amplitudenmittelwert anhand einer der betreffenden Gruppe zugeordneten Fitting-Funktion korrigiert wird. Bei einer solchen Vorgehensweise wird allen Spektralkomponenten der Gruppe der gleiche korrigierte Amplitudenwert zugeordnet.

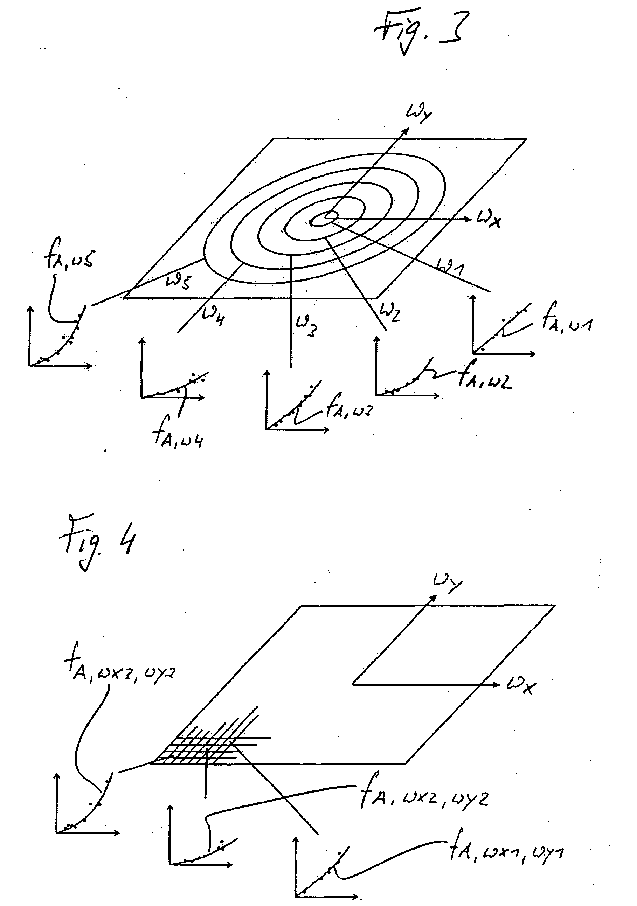

Die beiden vorstehend skizzierten unterschiedlichen Vorgehensweisen sind in den Figuren 3 und 4 noch einmal bildlich verdeutlicht. Figur 3 zeigt den Fall einer gruppenweisen Korrektur von Spektralkomponenten des Ablationsprofils, während Figur 4 den Fall einer einzelweisen Korrektur zeigt. Es versteht sich, dass beide Methoden miteinander kombiniert werden können, indem einzelne Spektrallinien individuell amplitudenkorrigiert werden, andere dagegen gruppenweise.

In Figur 3 sind fünf verschiedene Frequenzringe angedeutet, die in dem durch orthogonale Ortsfrequenzkomponenten ωₓ und ω_{y} aufgespannten zweidimensionalen Ortsfrequenzraum Orte gleichen Frequenzwerts darstellen. Diese Frequenzringe sind durch Frequenzwerte ω₁, ω₂, ω₃, ω₄, ω₅ charakterisiert. Jedem Frequenzring ist eine gesonderte Amplitudenkorrekturfunktion f_{A, ω1} bis F_{A}, _{ω5} zugeordnet, anhand der ein für die Spektrallinien des betreffenden Frequenzrings ermittelter Amplitudenmittelwert korrigiert wird. Die Korrektur erfolgt einfach in der Weise, dass der betreffende (gemittelte) spektrale Amplitudenwert des Ablationsprofils des Auges 16 als Ist-Amplitudenwert genommen wird und anhand der zugehörigen Fitting-Funktion der zur Erzielung dieses Ist-Amplitudenwerts benötigte Soll-Amplitudenwert ermittelt wird.

In Figur 4 dagegen ist verschiedenen Spektralkomponenten jeweils individuell eine gesonderte Fitting-Funktion zugeordnet, wobei Frequenzwert und Raumrichtung der einzelnen Spektralkomponenten durch die jeweiligen Werte der Ortsfrequenzkomponenten ωₓ, ω_{y} definiert sind. Beispielhaft sind (nichtlineare) Fitting-Funktionen f_{A, ωx1}, _{ωy1} bis f_{A, ωx3, ωy3} skizziert, die Spektralkomponenten mit den durch die Paare (ωₓ₁, ω_{y1}), (ωₓ₂, ω_{y2}), (ωₓ₃, ω_{y3}) definierten Ortsfrequenzen zugeordnet sind.
Es wurde bereits darauf hingewiesen, dass Frequenzkopplungen zwischen verschiedenen Ortsfrequenzen des Soll-Ablationsprofils auftreten können, die dazu führen, dass die Ist-Amplitude bei einer Ortsfrequenz nicht nur von der Soll-Amplitude bei dieser Ortsfrequenz abhängt, sondern auch von der Soll-Amplitude bei einer oder mehreren anderen Ortsfrequenzen. Für eine möglichst genaue Korrektur des Ablationsprofils eines zu behandelnden Auges empfiehlt es sich, in den Fitting-Funktionen solche Frequenzkopplungen widerzuspiegeln. Zur Ermittlung der Fitting-Funktionen werden deshalb vorzugsweise nicht nur Paare von Soll-Amplitude und Ist-Amplitude ein und derselben Ortsfrequenz betrachtet, sondern Tupel, die sich aus der Ist-Amplitude einer Ortsfrequenz, der Soll-Amplitude dieser Ortsfrequenz und der Soll-Amplitude einer oder mehrerer anderer Ortsfrequenzen zusammensetzen. Dies führt entsprechend zu mehrdimensionalen Fitting-Funktionen.

Es hat sich gezeigt, dass Frequenzkopplungen sich durch eine vergleichsweise hohe Streuung der Wertepaare von Ist-Amplitude und Soll-Amplitude bei einer gegebenen Ortsfrequenz bemerkbar machen. Um das Ausmaß der Frequenzkopplungen festzustellen, kann gemäß einer Ausführungsform zunächst eine Korrelationsanalyse der in der Patientendatenbasis vorhandenen Ist-Amplituden und Soll-Amplituden durchgeführt werden. Beispielsweise kann hierfür die Kreuzkorrelation der Ist-Amplituden verschiedener Patienten bei einer Ortsfrequenz und der Soll-Amplituden derselben Patienten bei dieser Ortsfrequenz und mehreren anderen Ortsfrequenzen berechnet werden. Ergebnis ist eine Kreuzkorrelationsmatrix, aus der ersehbar ist, wie stark die Ist-Amplitude bei der betreffenden einen Ortsfrequenz durch die Soll-Amplituden der anderen Ortsfrequenzen beeinflusst wird. Wird hierbei eine starke Beeinflussung durch eine oder mehrere andere Ortsfrequenzen festgestellt, kann dieser Einfluss durch entsprechende Berücksichtigung der Soll-Amplituden dieser anderen Ortsfrequenzen beim Funktionsfitting berücksichtigt werden. Zeigt dagegen die Korrelationsanalyse, dass der Einfluss der anderen Ortsfrequenzen gering oder vernachlässigbar ist, kann ein eindimensionales Funktionenfitting durchgeführt werden, wie weiter oben im Zusammenhang mit Figur 2 erläutert.

## Patentansprüche

1. Verfahren zur Ermittlung von Steuerinformationen für die Steuerung von auf die Hornhaut eines photorefraktiv zu behandelnden Auges (16) eingestrahlter Laserstrahlung (14), wobei bei diesem Verfahren ein durch Vermessung der optischen Eigenschaften des zu behandelnden Auges gewonnenes Hornhautablationsprofil anhand von Korrekturinformationen korrigiert wird und die Steuerinformationen auf Grundlage des so erzeugten korrigierten Ablationsprofils gebildet werden,
**dadurch gekennzeichnet, dass** das Ablationsprofil in ein Ortsfrequenzspektrum transformiert wird, dass sodann für verschiedene diskrete Spektralkomponenten des Ablationsprofils auf Grundlage gespeicherter Amplitudenkorrekturinformationen jeweils ein korrigierter Amplitudenwert ermittelt wird und dass zur Ermittlung des korrigierten Ablationsprofils anschließend das Ortsfrequenzspektrum mit den amplitudenkorrigierten Spektralkomponenten in den geometrischen Raumbereich zurücktransformiert wird, wobei die Amplitudenkorrekturinformationen für mehrere sich zumindest durch den Frequenzwert unterscheidende Ortsfrequenzen (ω₁, ω₂) jeweils einen vorab ermittelten Zusammenhang (f(A_{S, ω1}), f(A_{S, ω2})) zwischen Werten der Amplitude bei der betreffenden Ortsfrequenz und korrigierten Amplitudenwerten bei dieser Ortsfrequenz repräsentieren.

2. Verfahren nach dem Oberbegriff des Anspruchs 1, **dadurch gekennzeichnet, dass** das Ablationsprofil in ein Ortsfrequenzspektrum transformiert wird, dass sodann für verschiedene diskrete Spektralkomponenten des Ablationsprofils auf Grundlage gespeicherter Phasenkorrekturinformationen jeweils ein korrigierter Phasenwert ermittelt wird und dass zur Ermittlung des korrigierten Ablationsprofils anschließend das Ortsfrequenzspektrum mit den phasenkorrigierten Spektralkomponenten in den geometrischen Raumbereich zurücktransformiert wird, wobei die Phasenkorrekturinformationen für mehrere sich zumindest durch den Frequenzwert unterscheidende Ortsfrequenzen (ω₁, ω₂) jeweils einen vorab ermittelten Zusammenhang zwischen Werten der Phase bei der betreffenden Ortsfrequenz und korrigierten Phasenwerten bei dieser Ortsfrequenz repräsentieren.

3. Verfahren nach dem Oberbegriff des Anspruchs 1, **dadurch gekennzeichnet, dass** das Ablationsprofil in ein Ortsfrequenzspektrum transformiert wird, dass sodann für verschiedene diskrete Spektralkomponenten des Ablationsprofils auf Grundlage gespeicherter Realteilkorrekturinformationen jeweils ein korrigierter Realteilwert ermittelt wird und dass zur Ermittlung des korrigierten Ablationsprofils anschließend das Ortsfrequenzspektrum mit den realteilkorrigierten Spektralkomponenten in den geometrischen Raumbereich zurücktransformiert wird, wobei die Realteilkorrekturinformationen für mehrere sich zumindest durch den Frequenzwert unterscheidende Ortsfrequenzen (ω₁, ω₂) jeweils einen vorab ermittelten Zusammenhang zwischen Werten des Realteils bei der betreffenden Ortsfrequenz und korrigierten Realteilwerten bei dieser Ortsfrequenz repräsentieren.

4. Verfahren nach dem Oberbegriff des Anspruchs 1, **dadurch gekennzeichnet, dass** das Ablationsprofil in ein Ortsfrequenzspektrum transformiert wird, dass sodann für verschiedene diskrete Spektralkomponenten des Ablationsprofils auf Grundlage gespeicherter Imaginärteilkorrekturinformationen jeweils ein korrigierter Imaginärteilwert ermittelt wird und dass zur Ermittlung des korrigierten Ablationsprofils anschließend das Ortsfrequenzspektrum mit den imaginärteilkorrigierten Spektralkomponenten in den geometrischen Raumbereich zurücktransformiert wird, wobei die Imaginärteilkorrekturinformationen für mehrere sich zumindest durch den Frequenzwert unterscheidende Ortsfrequenzen (ω₁, ω₂) jeweils einen vorab ermittelten Zusammenhang zwischen Werten des Imaginärteils bei der betreffenden Ortsfrequenz und korrigierten Imaginärteilwerten bei dieser Ortsfrequenz repräsentieren.

5. Verfahren nach einem der vorhergehenden Ansprüche,
**dadurch gekennzeichnet, dass** für mehrere sich hinsichtlich Frequenzwert oder/und Raumrichtung unterscheidende Spektralkomponenten ((ωₓ₁, ω_{y1}), (ωₓ₂, ω_{y2}), (ωₓ₃, ω_{y3})) des Ablationsprofils jeweils individuell eine amplituden- oder/und phasen- oder/und realteil- oder/und imaginärteilkorrigierte Spektralkomponente auf Grundlage der Amplituden- oder/und Phasen- oder/und Realteil- oder/und Imaginärteilkorrekturinformationen ermittelt wird.

6. Verfahren nach einem der vorhergehenden Ansprüche,
**dadurch gekennzeichnet, dass** für eine Gruppe von Spektralkomponenten gleichen Frequenzwerts (ω₁, ω₂, ω₃, ω₄, ω₅), jedoch unterschiedlicher Raumrichtung des Ablationsprofils eine gemeinsame amplituden- oder/und phasen- oder/und realteil- oder/und imaginärteilkorrigierte Spektralkomponente auf Grundlage der Amplituden- oder/und Phasen- oder/und Realteil- oder/und Imaginärteilkorrekturinformationen ermittelt wird.

7. Verfahren nach Anspruch 6,
**dadurch gekennzeichnet, dass** die Amplituden oder/und Phasen oder/und Realteile oder/und Imaginärteile der Spektralkomponenten der Gruppe gemittelt werden und die gemeinsame amplituden- oder/und phasen- oder/und realteil- oder/und imaginärteilkorrigierte Spektralkomponente abhängig von dem Amplituden-, Phasen-, Realteil- bzw. Imaginärteilmittelwert ermittelt wird.

8. Verfahren nach einem der vorhergehenden Ansprüche,
**dadurch gekennzeichnet, dass** mindestens einer der durch die Amplitudenkorrekturinformationen repräsentierten Zusammenhänge oder/und mindestens einer der durch die Phasenkorrekturinformationen repräsentierten Zusammenhänge die korrigierten Amplituden- bzw. Phasenwerte bei der betreffenden Ortsfrequenz auch abhängig vom Wert der Amplitude bzw. Phase bei mindestens einer anderen Ortsfrequenz angibt.

9. Verfahren nach einem der vorhergehenden Ansprüche,
**dadurch gekennzeichnet, dass** mindestens einer der durch die Realteilkorrekturinformationen repräsentierten Zusammenhänge oder/und mindestens einer der durch die Imaginärteilkorrekturinformationen repräsentierten Zusammenhänge die korrigierten Realteil- bzw. Imaginärteilwerte bei der betreffenden Ortsfrequenz auch abhängig vom Wert des Realteils bzw. des Imaginärteils bei mindestens einer anderen Ortsfrequenz angibt.

10. Verfahren nach einem der vorhergehenden Ansprüche,
**dadurch gekennzeichnet, dass** das Ortsfrequenzspektrum des Ablationsprofils durch Fourier-Analyse, insbesondere komplexe Fourier-Analyse, ermittelt wird.

11. Verfahren zur Erzeugung von Amplituden- oder/und Phasen- oder/und Realteil- oder/und Imaginärteilkorrekturinformationen zur Verwendung bei dem Verfahren nach einem der vorhergehenden Ansprüche,
**dadurch gekennzeichnet, dass** eine Vielzahl von Soll-Ablationsprofilen und eine Vielzahl zugehöriger Ist-Ablationsprofile jeweils in ein Ortsfrequenzspektrum transformiert werden, dass anhand der Soll- und der Ist-Ortsfrequenzspektren für mehrere sich hinsichtlich Frequenzwert oder/und Raumrichtung unterscheidende diskrete Ortsfrequenzen jeweils ein funktionaler Zusammenhang für die Abhängigkeit der Ist-Amplitude oder/und der Ist-Phase oder/und des Ist-Realteils oder/und des Ist-Imaginärteils bei der betreffenden Ortsfrequenz von der Soll-Amplitude bzw. der Soll-Phase bzw. dem Soll-Realteil bzw. dem Soll-Imaginärteil bei dieser Ortsfrequenz ermittelt wird und dass die Amplituden- oder/und Phasen- oder/und Realteil- oder/und Imaginärteilkorrekturinformationen von Daten gebildet werden, welche die ermittelten funktionalen Zusammenhänge beschreiben.

12. Verfahren nach Anspruch 11,
**dadurch gekennzeichnet, dass** die funktionalen Zusammenhänge jeweils durch Approximation mittels einer linearen oder nichtlinearen Funktion ermittelt werden.

13. Verfahren nach Anspruch 11 oder 12,
**dadurch gekennzeichnet, dass** bei der Ermittlung der funktionalen Zusammenhänge die Abhängigkeit der Ist-Amplitude oder/und der Ist-Phase oder/und des Ist-Realteils oder/und des Ist-Imaginärteils mindestens einer Ortsfrequenz von der Soll-Amplitude bzw. der Soll-Phase bzw. dem Soll-Realteil bzw. dem Soll-Imaginärteil mindestens einer anderen Ortsfrequenz untersucht wird, derart, dass mindestens ein Teil der funktionalen Zusammenhänge die Ist-Amplitude oder/und die Ist-Phase oder/und den Ist-Realteil oder/und den Ist-Imaginärteil bei der jeweils betreffenden Ortsfrequenz auch in Abhängigkeit von der Soll-Amplitude bzw. der Soll-Phase bzw. dem Soll-Realteil bzw. dem Soll-Imaginärteil bei mindestens einer anderen Ortsfrequenz angibt.

## Claims

1. A method for determining control information for controlling laser radiation (14) irradiated onto the cornea of an eye (16) to be treated photorefractively, in which method a corneal ablation profile which is obtained by measuring the optical properties of the eye to be treated is corrected by means of correction information, and the control information is formed on the basis of the ablation profile generated in this manner,
**characterised in that** the ablation profile is transformed into a spatial frequency spectrum, **in that** a corrected amplitude value each is then determined for various discrete spectral components of the ablation profile on the basis of stored amplitude correction information, and **in that** the spatial frequency spectrum including the amplitude-corrected spectral components is subsequently re-transformed into the geometric spatial domain for determining the corrected ablation profile, wherein the amplitude correction information for several spatial frequencies (ω₁, ω₂), which differ at least in terms of frequency value, represent a relationship in each case (f(A_{s,ω1}), f(A_{s,ω2})), which has been determined in advance between amplitude values at the relevant spatial frequency and corrected amplitude values at this spatial frequency.

2. The method according to the preamble of Claim 1,
**characterised in that** the ablation profile is transformed into a spatial frequency spectrum, **in that** a corrected phase value each is then determined for various discrete spectral components of the ablation profile on the basis of stored phase correction information, and **in that** the spatial frequency spectrum including the phase-corrected spectral components is subsequently re-transformed into the geometric spatial domain for determining the corrected ablation profile, wherein the phase correction information for several spatial frequencies (ω₁, ω₂), which differ at least in terms of frequency value, represent a relationship in each case, which has been determined in advance between phase values at the relevant spatial frequency and corrected phase values at this spatial frequency.

3. The method according to the preamble of Claim 1,
**characterised in that** the ablation profile is transformed into a spatial frequency spectrum, **in that** a corrected real part value each is then determined for various discrete spectral components of the ablation profile on the basis of stored real part correction information, and **in that** the spatial frequency spectrum including the real part-correct spectral components is then re-transformed into the geometric spatial domain for determining the corrected ablation profile, wherein the real part correction information for several spatial frequencies (ω₁, ω₂), which differ at least in terms of frequency value, represent a relationship in each case, which has been determined in advance between values of the real part at the relevant spatial frequency and corrected values of the real part at this spatial frequency.

4. The method according to the preamble of Claim 1,
**characterised in that** the ablation profile is transformed into a spatial frequency spectrum, **in that** a corrected imaginary part value each is subsequently determined for various discrete spectral components on the basis of stored imaginary part correction information, and **in that** the spatial frequency spectrum including the imaginary part-corrected spectral components is subsequently re-transformed into the geometric spatial domain, wherein the imaginary part correction information for several spatial frequencies (ω₁, ω₂), which differ at least in terms of frequency value, represent a relationship in each case, which has been determined in advance between values of the real part at the relevant spatial frequency and corrected values of the imaginary part at this spatial frequency.

5. The method according to one of the previous claims,
**characterised in that** an amplitude-corrected or/and phase-corrected or/and real part-corrected or/and imaginary part-corrected spectral component each is determined individually on the basis of the amplitude or/and phase or/and real part or/and imaginary part correction information for several spectral components (ωₓ₁, ω_{y}), (ωₓ₂, ω_{y2}), (ωₓ₃, ω_{y3}) of the ablation profile, which differ in terms of frequency value or/and spatial direction.

6. The method according to one of the previous claims,
**characterised in that** a common amplitude-corrected or/and phase-corrected or/and real part-corrected or/and imaginary part-corrected spectral component is determined on the basis of the amplitude or/and phase or/and real part or/and imaginary part correction information for a group of spectral components of the ablation profile of the same frequency value (ω₁, ω₂, ω₃, ω₄, ω₅) but of different spatial direction.

7. The method according to Claim 6,
**characterised in that characterised in that** the amplitudes or/and phases or/and real parts or/and imaginary parts of the spectral components of the group are averaged and the common amplitude-corrected or/and phase-corrected or/and real part-corrected or/and imaginary part-corrected spectral component is determined as a function of the mean value of amplitude, phase, real part or imaginary part, respectively.

8. The method according to one of the previous claims,
**characterised in that** at least one of the relationships represented by the real part correction information or/and at least one of the relationships represented by the imaginary part correction information specifies/specify the corrected real part or imaginary part values, respectively, at the relevant spatial frequency also as a function of the amplitude value or of phase value, respectively, at at least one other spatial frequency.

9. The method according to one of the previous claims,
**characterised in that** at least one of the relationships represented by the real part correction information or/and at least one of the relationships represented by the imaginary part correction information specifies/specify the corrected real part or imaginary part values, respectively, at the relevant spatial frequency also as a function of the value of the real part or of value of the imaginary part, respectively, at at least one other spatial frequency

10. The method according to one of the previous claims,
**characterised in that** the spatial frequency spectrum of the ablation profile is determined by Fourier analysis, in particular complex Fourier analysis.

11. A method for generating amplitude or/and phase or/and real part or/and imaginary part correction information to be used in the method according to one of the previous claims,
**characterised in that** a plurality of set ablation profiles and a plurality of associated actual ablation profiles each are transformed into a spatial frequency spectrum, **in that** a functional relationship for the dependency of the actual amplitude or/and the actual phase or/and the actual real part or/and the actual imaginary part at the relevant spatial frequency from the set amplitude, or the set phase, or the set real part, or the set imaginary part, respectively, at this spatial frequency is determined with reference to the set and the actual spatial frequency spectra for several discrete spatial frequencies which differ in terms of frequency value or/and spatial direction, and **in that** the amplitude or/and phase or/and real part or/and imaginary part correction information is formed by data which describe the determined functional relationships.

12. The method according the Claim 11,
**characterised in that** the functional relationships are determined by approximation by means of a linear or a non-linear function.

13. The method according the Claim 11 or 12,
**characterised in that** in the determination of the functional relationships the dependency of the actual amplitude or/and the actual phase or/and the actual real part or/and the actual imaginary part of at least one spatial frequency from the set amplitude, or the set phase, or the set real part, or the set imaginary part, respectively, of at least one other spatial frequency is examined in such a manner that at least a portion of the functional relationships specifies the actual amplitude or/and the actual phase or/and the actual real part or/and the actual imaginary part at the relevant spatial frequency also as a function of the set amplitude, or the set phase, or the set real part, or the set imaginary part, respectively, at at least one other spatial frequency.

## Revendications

1. Procédé pour déterminer des informations de commande pour piloter un faisceau laser (14) dirigé sur la cornée d'un oeil (16) à traiter par photoréfraction, ce procédé consistant à corriger, sur la base d'informations correctives, un profil d'ablation de cornée obtenu par mesure des propriétés optiques de l'oeil à traiter, et les informations de commande étant formées sur la base du profil d'ablation corrigé ainsi obtenu,
**caractérisé en ce que** le profil d'ablation est transformé en un spectre de fréquence locale, qu'une valeur d'amplitude corrigée est ensuite déterminée pour différents éléments de spectre discrets du profil d'ablation sur la base d'informations correctives d'amplitude mémorisées et que le spectre de fréquence locale est ensuite retransformé dans l'espace géométrique avec les éléments de spectre à amplitude corrigée pour déterminer le profil d'ablation corrigé, les informations correctives d'amplitude représentant, pour plusieurs fréquences locales (ω₁, ω₂) se différenciant au moins par la valeur de fréquence, une corrélation (f(Aₛ, ω₁), f(Aₛ, ω₂)) définie par avance entre des valeurs d'amplitude pour la fréquence locale concernée et des valeurs de l'amplitude corrigées pour cette fréquence locale.

2. Procédé selon le préambule de la revendication 1, **caractérisé en ce que** le profil d'ablation est transformé en un spectre de fréquence locale, qu'une valeur de phase corrigée est ensuite déterminée pour différents éléments de spectre discrets du profil d'ablation sur la base d'informations correctives de phase mémorisées et que le spectre de fréquence locale est finalement retransformé dans l'espace géométrique avec les éléments de spectre à phase corrigée pour déterminer le profil d'ablation corrigé, les informations correctives de phase représentant, pour plusieurs fréquences locales (ω₁, ω₂) se différenciant au moins par la valeur de fréquence, une corrélation définie par avance entre des valeurs de la phase pour la fréquence locale concernée et des valeurs de phase corrigées pour cette fréquence locale.

3. Procédé selon le préambule de la revendication 1, **caractérisé en ce que** le profil d'ablation est transformé en un spectre de fréquence locale, qu'une valeur de partie réelle corrigée est ensuite déterminée pour différents éléments de spectre discrets du profil d'ablation sur la base d'informations correctives de partie réelle mémorisées et que le spectre de fréquence locale est finalement retransformé dans l'espace géométrique avec les éléments de spectre à partie réelle corrigée pour déterminer le profil d'ablation corrigé, les informations correctives de partie réelle représentant, pour plusieurs fréquences locales (ω₁, ω₂) se différenciant au moins par la valeur de fréquence, une corrélation définie par avance entre des valeurs de la partie réelle pour la fréquence locale concernée et des valeurs de partie réelle corrigées pour cette fréquence locale.

4. Procédé selon le préambule de la revendication 1, **caractérisé en ce que** le profil d'ablation est transformé en un spectre de fréquence locale, qu'une valeur de partie imaginaire corrigée est ensuite déterminée pour différents éléments de spectre discrets du profil d'ablation sur la base d'informations correctives de partie imaginaire mémorisées et que le spectre de fréquence locale est finalement retransformé dans l'espace géométrique avec les éléments de spectre à partie imaginaire corrigée pour déterminer le profil d'ablation corrigé, les informations correctives de partie imaginaire représentant, pour plusieurs fréquences locales (ω₁, ω₂) se différenciant au moins par la valeur de fréquence, une corrélation définie par avance entre des valeurs de la partie imaginaire pour la fréquence locale concernée et des valeurs de partie imaginaire corrigées pour cette fréquence locale.

5. Procédé selon l'une des revendications précédentes,
**caractérisé en ce qu'**un élément de spectre à amplitude ou/et à phase ou/et à partie réelle ou/et à partie imaginaire corrigées est respectivement déterminé individuellement pour plusieurs composants de spectre ((ωₓ₁, ω_{y1}), (ωₓ₂, ω_{y2}), (ωₓ₃, ω_{y3})) se différenciant du point de vue de la valeur de fréquence ou/et de la direction spatiale du profil d'ablation sur la base d'informations correctives d'amplitude, ou/et de phase ou/et de partie réelle ou/et de partie imaginaire.

6. Procédé selon l'une des revendications précédentes,
**caractérisé en ce qu'**un élément de spectre à amplitude ou/et à phase ou/et à partie réelle ou/et à partie imaginaire est déterminé pour un groupe d'éléments de spectre de valeur de fréquence (ω₁, ω₂, ω₃, ω₄, ω₅) identique mais de direction spatiale différente du profil d'ablation sur la base d'informations correctives d'amplitude ou/et de phase ou/et de partie réelle ou/et de partie imaginaire.

7. Procédé selon la revendication 6,
**caractérisé en ce que** les amplitudes ou/et les phases ou/et les parties réelles ou/et les parties imaginaires des composants de spectre du groupe sont moyennées et le composant de spectre à amplitude ou/et à phase ou/et à partie réelle ou/et à partie imaginaire commun est déterminé en fonction de la valeur moyenne d'amplitude, de phase, de partie réelle ou/et de partie imaginaire.

8. Procédé selon l'une des revendications précédentes,
**caractérisé en ce qu'**au moins une des corrélations représentées par les informations correctives d'amplitude ou/et au moins une des corrélations représentées par les informations correctives de phase indiquent les valeurs d'amplitude ou de phase corrigées pour la fréquence locale concernée également en fonction de la valeur d'amplitude ou/et de phase pour au moins une autre fréquence locale.

9. Procédé selon l'une des revendications précédentes,
**caractérisé en ce qu'**au moins une des corrélations représentées par les informations correctives de partie réelle ou/et au moins une des corrélations représentées par les informations correctives de partie imaginaire indiquent les valeurs de partie réelle ou/et de partie imaginaire corrigées pour la fréquence locale concernée également en fonction de la valeur de partie réelle ou/et de partie imaginaire pour au moins une autre fréquence locale.

10. Procédé selon l'une des revendications précédentes,
**caractérisé en ce que** le spectre de fréquence locale du profil d'ablation est déterminé par analyse de Fourier, plus particulièrement par analyse complexe de Fourier.

11. Procédé pour générer des informations correctives d'amplitude ou/et de phase ou/et de partie réelle ou/et de partie imaginaire à utiliser lors du procédé selon l'une des revendications précédentes,
**caractérisé en ce qu'**un grand nombre de profils d'ablation théoriques et un grand nombre de profils d'ablation réels connexes sont respectivement transformés en un spectre de fréquence locale, **en ce qu'**il est, sur la base des spectres de fréquence locale théoriques et réels pour plusieurs fréquences locales discrètes se différenciant du point de vue de la valeur de fréquence ou/et de la direction spatiale, respectivement déterminé une corrélation fonctionnelle pour la dépendance de l'amplitude réelle ou/et de la phase réelle ou/et de la partie réelle réelle ou/et de la partie imaginaire réelle pour la fréquence locale concernée à l'égard de l'amplitude théorique ou/et de la phase théorique ou/et de la partie réelle théorique ou/et de la partie imaginaire théorique pour cette fréquence locale et **en ce que** les informations correctives d'amplitude ou/et de phase ou/et de partie réelle ou/et de partie imaginaire sont formées par des données décrivant les corrélations fonctionnelles déterminées.

12. Procédé selon la revendication 11,
**caractérisé en ce que** les corrélations fonctionnelles sont respectivement déterminées par approximation au moyen d'une fonction linéaire ou non-linéaire.

13. Procédé selon la revendication 11 ou 12,
**caractérisé en ce que** lors de la détermination des corrélations fonctionnelles, la dépendance de l'amplitude réelle ou/et de la phase réelle ou/et de la partie réelle réelle ou/et de la partie imaginaire réelle d'au moins une fréquence locale à l'égard de l'amplitude théorique ou/et de la phase théorique ou/et de la partie réelle théorique et/ou de la partie imaginaire théorique d'au moins une autre fréquence locale, est examinée de telle sorte qu'au moins une partie des corrélations fonctionnelles indique l'amplitude réelle ou/et la phase réelle ou/et la partie réelle réelle ou/et la partie imaginaire réelle pour la fréquence locale concernée également en fonction de l'amplitude théorique et/ou de la phase théorique et/ou de la partie réelle théorique et/ou de la partie imaginaire théorique pour au moins une autre fréquence locale.
